# EUROPEAN PATENT SPECIFICATION

(11) **EP 3 180 064 B1**
(45) Date of publication and mention of the grant of the patent: **22.04.2020**
(21) Application number: 14899786.9
(22) Date of filing: 15.08.2014
(51) Int. Cl.: A61M 29/00, A61M 25/06, A61M 25/00

(54) **EXPANDABLE SHEATH AND SYSTEM FOR INTRAVASCULAR INSERTION OF A MEDICAL IMPLEMENT USING THE SAME**
EXPANDIERBARE HÜLLE UND SYSTEM ZUR INTRAVASKULÄREN EINFÜHRUNG EINER MEDIZINISCHEN VORRICHTUNG DAMIT
GAINE EXTENSIBLE ET SYSTÈME D'INTRODUCTION INTRAVASCULAIRE D'UN INSTRUMENT MÉDICAL L'UTILISANT

(43) Date of publication of application: 21.06.2017
(73) Proprietor: Al-Rashdan, Ibrahim Rashid, Safat 13110 (KW); Levit, Eran, Amherst, New Hampshire 03031 (US)
(72) Inventor: Al-Rashdan, Ibrahim Rashid, Safat 13110 (KW); Levit, Eran, Amherst, New Hampshire 03031 (US)
(74) Representative: Abraham, Richard
(86) International application number: PCT/US2014/051237
(87) International publication number: WO 2016/024989

(56) References cited:
- US-A- 5 885 217
- US-A1- 2003 144 628
- US-A1- 2003 216 771
- US-A1- 2006 025 749
- US-A1- 2008 183 163
- US-A1- 2008 243 081
- US-A1- 2008 306 442
- US-A1- 2010 094 392
- US-A1- 2011 152 763
- US-A1- 2011 276 002
- US-A1- 2013 317 439

## Description

### TECHNICAL FIELD

The present invention relates to medical devices, and particularly to an expandable sheath for insertion of an arterial catheter.

### BACKGROUND ART

Less invasive medical procedures for various operations are, of course, highly desirable, as they result in less trauma for the patient, faster healing, less time spent in the hospital, less expense to the patient, and the ability for the patient to return to his or her work or normal lifestyle more rapidly. This is particularly true in cardiac procedures. If a surgical procedure can be accomplished without an open chest, or open-heart, surgery, such less invasive practices are much more desirable.

An example of such is the opening of various coronary arteries that have become obstructed by cholesterol deposits. The correction of this condition originally required surgery to access the interiors of the arteries directly for curettage. More recently, such arterial deposits have been flattened or pressed against, or into, the walls of the arteries by the balloon angioplasty procedure, wherein an inflatable device is inserted into the artery and expanded to widen the artery for proper blood flow. Accessing the femoral artery in one of the thighs of the patient, and working a catheter through the artery until reaching the desired target area in the coronary artery conventionally accomplish this procedure. The balloon is expanded to widen the artery when the balloon reaches the target area. While this procedure is clearly less invasive than open chest surgery, the length of the catheter and sheath required, as well as the delicate manipulation of the catheter and sheath through such a relatively long pathway, make this a relatively intricate procedure. Various other procedures, such as coronary angioplasty, coronary arteriography, cardiac catheterization, etc., also typically involve the insertion of catheters, guides, and the like through an introducer sheath in the femoral artery.

More recently, there has been interest in developing techniques for access of coronary arteries through the radial artery in the wrist or lower arm of the patient. This procedure has advantages, including the far shorter distance required for manipulation of the distal end of the catheter from the entry site to the coronary artery. This also generally results in fewer traumas to the patient, as the percutaneous opening need not be so large when accessing the smaller radial artery in comparison to the femoral artery. However, the radial artery is a smaller diameter vessel than the femoral artery. Generally, it is desirable to keep the puncture of the vessel as small as possible. Nevertheless, coronary procedures often require the use of larger diameter guides, catheters, etc., than desirable in the radial artery.

Known prior art devices are disclosed in US2013317439, US20100094392, US2011152763, US2008306442 and US2008243081.

The medical profession uses a measurement system known as the French system for the diameters of these various sheaths and catheters. In the French system, one French is equal to one third of a millimeter, i.e., 3F = 1.0 mm, with a linear correspondence between the 20 French and metric dimensions. Typical guide and sheath diameters for use in a radial artery intervention procedure might be 2.00 mm or 6 French for the sheath, but the use of a larger 2.33 mm or 7 French guide is desirable in order to provide sufficient volume within the guide for the catheters and guides used for coronary procedures.

Thus, an expandable sheath and system for intravascular insertion of a medical implement using the same solving the aforementioned problems is desired.

### DISCLOSURE OF INVENTION

In accordance with a first aspect the present invention there is provided an expandable sheath as defined in appendant claim 1, to which reference should now be made. In accordance with further aspects of the present invention, there is also provided a system for intravascular insertion of a medical implement and a kit as defined in appendant independent claims 3 and 9 respectively. Embodiments of the present invention are defined in the appendant dependent claims.

The system for intravascular insertion of a medical implement of the present invention includes a cannula for entering a lumen of a vascular vessel of a patient, a guide wire insertable into the lumen, an introducer and dilator adapted to follow the guide wire into the lumen, an expandable sheath positioned on the introducer and dilator, with the expandable sheath being adapted to be positioned in the vessel, and an extension collar. The medical implement is received within the vessel by passing through the sheath upon separating the introducer and dilator therefrom.

The expandable sheath of the present invention is formed as an elongate flexible tubular member adapted for placement percutaneously within the vessel of the patient. The tubular member is preferably formed of a polymer material and includes a wall of substantially uniform thickness. The wall has an outer surface and an inner surface, with the wall having a first portion, a transitional portion and a second portion respectively having first, transitional and second inner diameters associated therewith. The first inner diameter is greater than the second inner diameter, and the transitional inner diameter is less than the first inner diameter and greater than the second inner diameter. In one embodiment, upon placement in the vessel, the sheath is adapted to slidably receive intravascular devices therein. In one embodiment, the tubular member includes a linear array of perforations formed therethrough, with the linear array of perforations extending from the second portion to the transitional portion, such that the linear array of perforations allow the second and transitional portions to expand.

These and other features of the present invention will become readily apparent upon further review of the following specification and drawings.

### BRIEF DESCRIPTION OF DRAWINGS

Fig. 1 is a perspective view of an expandable sheath and system for intravascular insertion of a medical implement using the same according to the present invention.
Fig. 2 is a partially exploded perspective view of an expandable sheath and system for intravascular insertion of a medical implement using the same according to the present invention.
Fig. 3 is a further partially exploded perspective view of an expandable sheath and system for intravascular insertion of a medical implement using the same according to the present invention.
Fig. 4 is a perspective view of the expandable sheath and system for intravascular insertion of a medical implement using the same.
Fig. 5A is an elevational view of an alternative embodiment of the expandable sheath and system for intravascular insertion of a medical implement using the same.
Fig. 5B is an enlarged view of the portion 5B of the expandable sheath and system for intravascular insertion of a medical implement using the same of Fig. 5A.
Fig. 5C is an enlarged view of the portion 5C of the expandable sheath and system for intravascular insertion of a medical implement using the same of Fig. 5A.
Fig. 6A is an elevational view of another alternative embodiment of the expandable sheath and system for intravascular insertion of a medical implement using the same.
Fig. 6B is cross-sectional view taken alone sectional cut line 6B-6B of Fig. 6A.
Fig. 7 illustrates a kit including the expandable sheath and system for intravascular insertion of a medical implement using the same.
Fig. 8 illustrates the kit of Fig. 7 provided in sterile packaging.

Similar reference characters denote corresponding features consistently throughout the attached drawings.

### BEST MODES FOR CARRYING OUT THE INVENTION

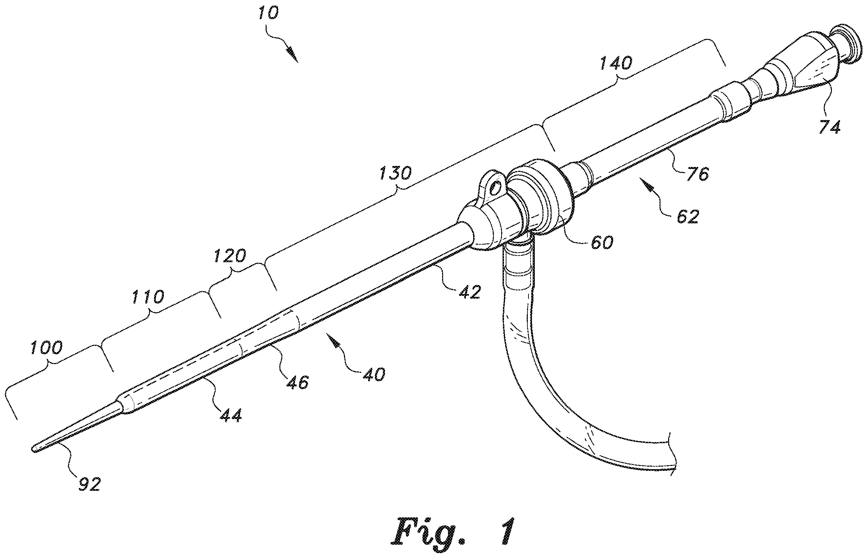

The expandable sheath and system for intravascular insertion of a medical implement, as shown in Fig. 1, generally identified as 10, includes an introducer and dilator 62 having a proximal end 74, a collar 76, a sheath 40, and a hub 60. The sheath 40 has a proximal end 42, a transitional area 46, and a distal end 44. The sheath is shown disposed about the introducer and dilator 62, with the distal end 92 of introducer and dilator 62 shown extending from the distal end 44 of sheath 40. The hub 60 may include, or be connected with, vascular procedural specific components, such as a hemostasis valve, for example.

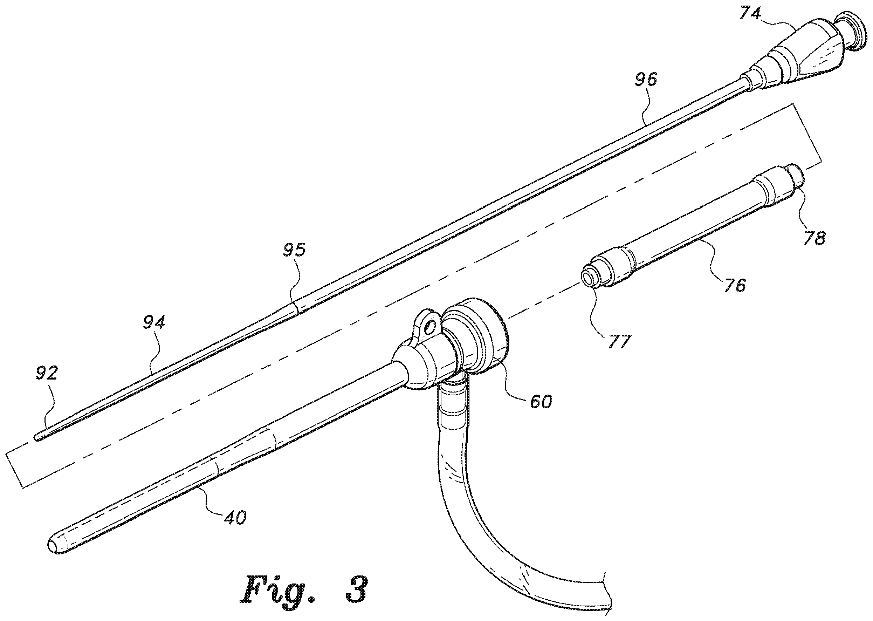

As shown in Figs. 1-3, the overall length of the system 10 is segmented into five general regions. Each region has a specific geometrical form, and function, discussed below. Section 100 represents the distal end 92 of introducer and dilator 62, section 110 includes the portion 94 of the introducer and dilator 62 (as shown in Fig. 3), upon which the distal end 44 of sheath 40 is mounted. Section 120 represents the transitional portion 95 of introducer and dilator 62, and the corresponding transitional section of 46 of sheath 40. Section 130 includes a part of portion 96 of introducer and dilator 62, and the proximal end 42 of the sheath, and hub 60 (connected to the proximal end 42). Section 140, includes the remaining portion 96 of the introducer and dilator 62, along with an extension collar 76.

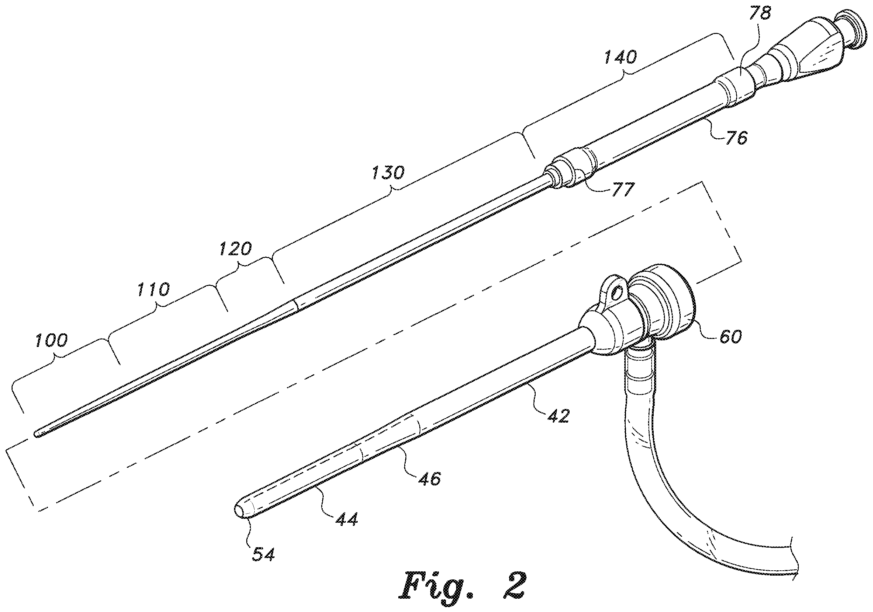

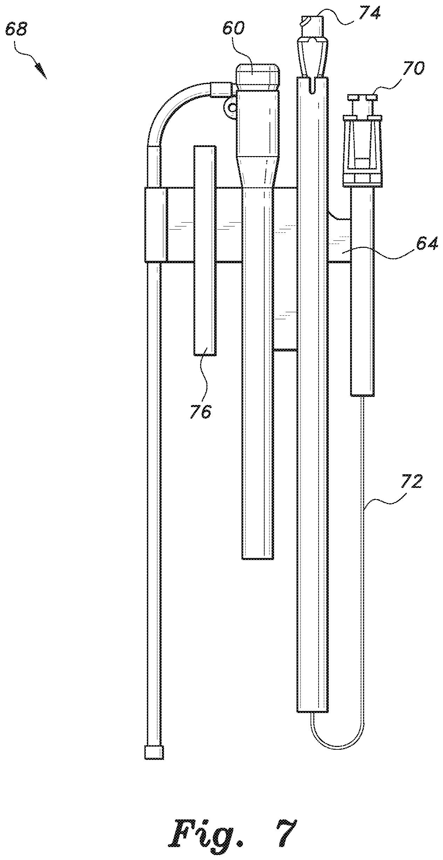

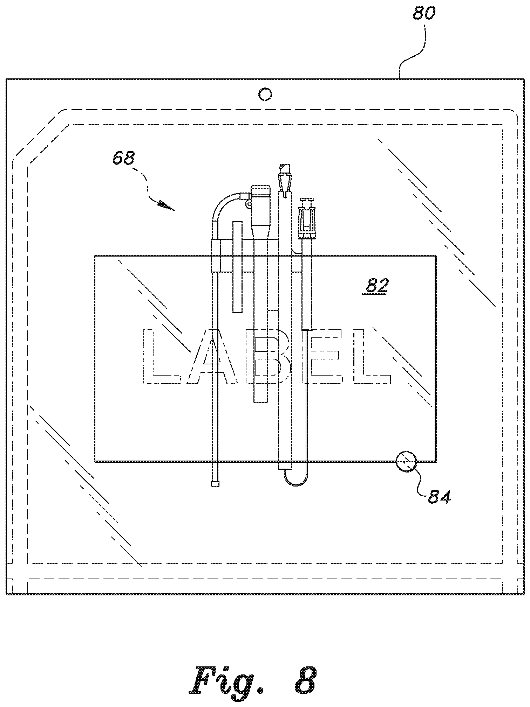

As shown in Figs. 7 and 8, a cannula 70 and guide wire 72 are provided for introduction into the lumen of a vascular member. Initially, the cannula 70 is used to puncture the skin, tissue, and vascular vessel, penetrating into the lumen. The guide wire 72 is subsequently introduced via the cannula 70 into the vessel, and remains traversing the skin, tissue, and vessel wall upon removal of the cannula 70. The system 10 is thusly placed about the guide wire 72 (via the hollow interior of the introducer and dilator 62), and extended into the vessel lumen. The distal end 92 of the introducer and dilator 62 is tapered so as to gradually enter the aperture produced by the cannula 70, increasing the aperture as the introducer and dilator 62 proceeds into the lumen. As best shown in Fig. 2, the distal end of the sheath 44 includes a tapered or rounded tip 54 that follows the distal end 92 of the introducer and dilator 62, gradually increasing the size of the aperture leading into the vessel without causing any undue harm or injury to the skin, tissue, and vessel.

The proximal end 42 of the sheath 40 may be color coded, or have indicia markings to indicate orientation and depth of penetration into the vessel. Once the desired penetration depth has been achieved, the introducer and dilator 62 is removed, and another implement, such as a catheter, for example, may be inserted into the vessel via the sheath 40. The characteristics of the sheath 40 allow the catheter to enter into the vessel without causing any undue harm or injury to the vessel. The sheath 40 has, at its proximal end 42, a first inner diameter, and at its distal end 42 a second inner diameter.

Generally, the first inner diameter is larger than the second inner diameter. For example, the first inner diameter may be 8 Fr., while the second inner diameter may be 6 Fr. Between the proximal end 42 and the distal end 44 is a gradual transitional section 46 that connects the first inner diameter to the second inner diameter through a smooth, decrease in radial direction. This allows, for example, a gradual sloping from the 8 Fr. to the 6 Fr. inner diameters of the sheath 40. The hub 60, also has an inner diameter of 8 Fr. in alignment with the inner diameter of the proximal end 42 of sheath 40. In operation, a medical practitioner is able to safely insert a medical implement, such as a balloon catheter of 6 Fr. diameter into the vessel via the sheath 40.

The introducer and dilator 62 has corresponding outer diameters with the inner diameters of the sheath 40, in accordance with the sections 100-140 discussed above. Beginning from the tapered distal end 92 of introducer and dilator 62, segment 110, or portion 94, has an outer diameter equal to the inner diameter of distal end 44 of sheath 40, for example, 6 Fr. The outer diameter of the transition segment 120, or portion 95, of introducer and dilator 62 matches the inner diameter of transition section 46 of sheath 40. Likewise, the outer diameter of segment 130, or portion 96, of introducer and dilator 62 is equal to the inner diameter of proximal end 42 of sheath 40.

Situated about the extension of portion 96 is collar 76. Collar 76 has a first end 77 that matingly engages the hub 60 of sheath 40. As shown in Fig. 3, collar 76 has a second end 78 that forms a stop for the end piece 74 of introducer and dilator 62. The collar 76 prevents the introducer and dilator 62 from being inserted further into the sheath 40 than the depth of penetration indicia of the sheath requires. More importantly, this is the case when medical implements, such as catheters having maximum diameters of 6 Fr., are being used for vascular procedures. Collar 76 preferably releasably locks with both end piece 74 of introducer and dilator 62 and with sheath hub 60. It should be understood that any suitable type of locking mechanism or releasable connector may be utilized. As an alternative, collar 76 may be replaced by a peel-away or tear-away collar, thus saving time in the conventional procedure, in which the dilator is removed, the collar is then removed, and then the dilator is re- inserted.

If the need arises for medical implements having a greater diameter, such as 7 Fr. or 8 Fr., for example, conventional prior art systems require a different sheath to be inserted, which requires the removal of the first sheath, possibly causing blood loss and other injury to the vessel and surrounding tissue. The sheath 40, however, avoids delay in catheter insertion due to repenetration of the introducer and dilator 62 and sheath 40. Sheath 40 includes structure that allows for the expansion of the second inner diameter, as well as the transitional diameter, so that the inner diameter of the sheath 40 is uniformly the same as the first inner diameter of the proximal end 42.

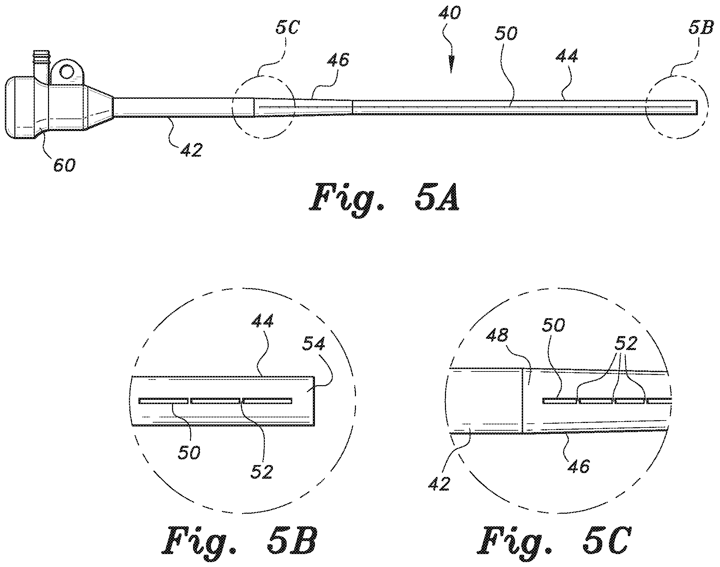

In the alternative embodiment of Figs. 5A-5C, sheath 40 has a linear array of perforations 50 formed therethrough. The perforations 50 extend from transition section 46 to near the tip of distal end 44. As best shown in the enlarged views of Figs. 5B and 5C, the perforations 50 are preferably linear, with each perforation 50 being separated from the adjacent perforation by a segment 52. The perforations 50 begin at the tip 54 without breaching the open end of distal end 44 (see Fig. 5B). Likewise, the perforations 50 end near the junction of the proximal end 42 and transition portion 46, such that that a space 48 prevents the perforations from encroaching into the proximal end 44. It should be understood that the linear perforations are shown for exemplary purposes only, and that the perforations may have any desired contouring and relative dimensions, such as circular, elliptical, etc. Additionally, as best shown in the comparison between Figs. 5B and 5C, the perforations 50 toward the distal end of the sheath 40 are preferably longer to allow for less resistance while introducing the larger portion of the dilator and to expand the sheath with less force.

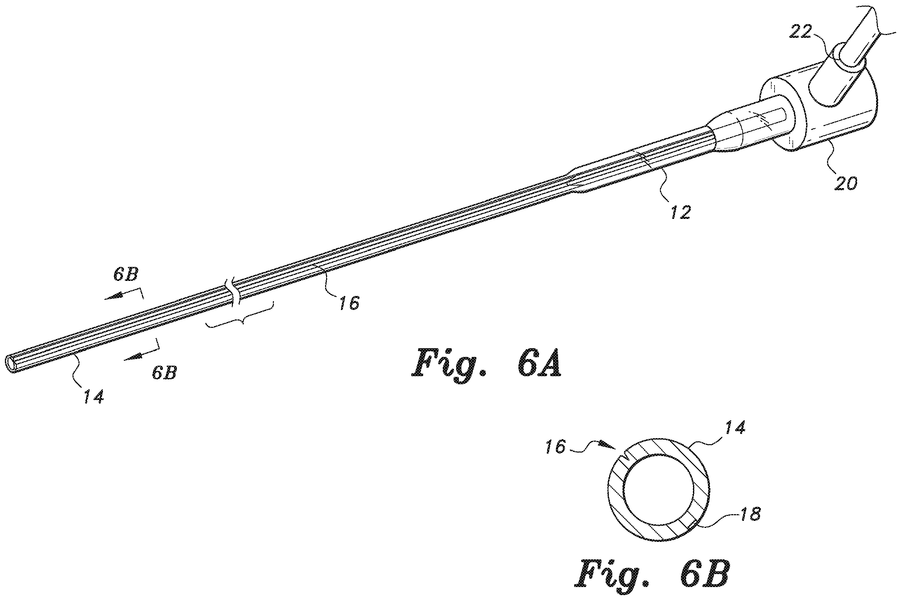

In the embodiment of Figs. 6A and 6B (taken along direction 6B-6B of Fig. 6A), the sheath 40 may be an elongated cylindrical tube having a large diameter portion 12 at the proximal end thereof (for example, with an inner diameter of 8 Fr.) with an elongated slit 16 extending longitudinally from the distal end of the sheath towards the large diameter portion. It will be understood that the slit 16 may extend for more than one-half the length of the sheath 40, and may extend up to 80-90% of the length of the sheath 40, extending from the smaller diameter portion 14 of sheath 40 (having a diameter of 6 Fr., for example). The slit 16 may be formed by any suitable method, such as, for example, by using a circular blade with grooves. When such a blade is rolled on the sheath, the grooves leave non-slit portions between the slits created by the circular blade. The slit portion 16 is preferably formed so that the edges of the slit are harmless to the arterial wall. In the formation example given above, the way the blade edges are formed, the sharp edges of the blade slit the tube and the slit edges are compressed by the non-sharpened edges and form non-traumatic edges on both sides of slit.

The slit 16 in portion 14 is kept tight to prevent bleeding. The slit 16 must avoid bleeding when sheath 40 is introduced into the artery and is used within its original dimensions (i.e., non-expanded) to deliver catheterization tools with the outside diameter smaller than the inner original diameter (i.e., non-expanded) of the sheath. Additionally, as shown in Fig. 6B, indicia 18 may also be formed on the sheath 40 to indicate orientation, penetration depth, and any other desired positional characteristic of the sheath 40. Indicia 18 may include markings, etchings, arrows, and/or color-coding along the entire length of sheath 40. Indicia 18 preferably is formed on the proximal end 42, so as to extend outside the radial artery (upon insertion), away from the patient. As an alternative to the pre-formed perforations 50 of Figs. 5A-5C or the pre-formed external slit 16 of Figs. 6A and 6B, the dilator could be provided with a blade or the like for creating a continuous internal slit as the sheath 40 is inserted thereon. Such a blade or the like mounted on dilator 78, preferably between sections 110 and 120, would create an internal slit, allowing for expansion as described above with regard to both perforations 50 and external slit 16.

The purpose of the perforations 50 (Figs. 5A-C) and slit 16 (Figs. 6A and 6B) is to provide the medical practitioner with the ability to utilize a single system for multiple sized catheters during an intravascular procedure. The sheath 40 in both embodiments is expandable, thus increasing the second inner diameter and the transitional diameter to match the first inner diameter; i.e., expanding the transition section 46 and distal end 44 so that the respective inner diameters match the larger inner diameter of the proximal end 42. In the example described above, the 6 Fr. distal diameter is expanded to match the 8 Fr. proximal diameter.

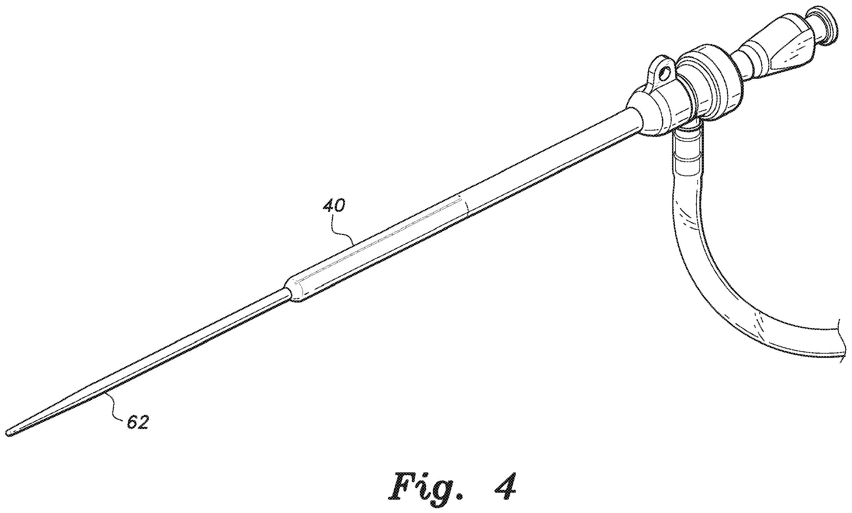

As best shown in Figs. 3 and 4, after the system 10 has been inserted into a vessel, the introducer and dilator 62 is withdrawn from sheath 40, the extension collar 76 is removed by disengaging the first end 77 from the hub 60. The introducer and dilator 62 is reinserted into the sheath 40 via hub 60, and is extended along the length of the segment 140 (i.e., the remaining part of portion 96). Upon reinsertion, the transitional portion 95 of introducer and dilator 62 forces the transitional section 46 and distal end 44 of sheath 40 radially outward so that the inner diameter is consistent throughout the sheath 40. The proximal end portion 96 of the introducer and dilator 62 has an outer diameter equal to the inner diameter of proximal end of the sheath 40, thus maintaining the expansion until the introducer and dilator 62 has traveled the full extent of the length through the five segments 100, 110, 120, 130, 140.

As shown in Fig. 4, the sheath 40 has a constant diameter externally, indicative of the internal diameter also being constant. In this particular example, the inner diameter has been expanded to 8 Fr., thereby allowing the medical practitioner to utilize implements that are greater than 6 Fr. in the sheath 40. Once the introducer and dilator 62 is removed, the expanded sheath allows the larger diameter implements to be inserted into the vascular vessel. Further, it is clear that the introducer and dilator 62 is a single element used in the system for both guiding the sheath 40 into the vessel, as well as causing the sheath 40 to be expanded.

Additionally, the first end 77 of collar 76 is designed and configured to matingly engage the hub 60 of sheath 40. The mating engagement may be accomplished in any suitable manner, including, but not limited to, friction fit, threads, twist lock, key and channel, dovetail, etc. Likewise, the second end 78 of the collar 76, which stops the forward progression of the introducer and dilator 62 into the sheath 40, may also be a mating engagement, similar to the first end and 77 with hub 60, forming an abutment for the end 74 to stop against.

In the embodiment of Figs. 5A-C, sheath 40 may be made from any suitable polymer or plastic material that has sufficient plasticity so that, upon the expansion of the perforations 50, the sections 52 will stretch to accommodate the larger diameter, but upon removal of the introducer and dilator 62, sections 52 will not return to the original shape. Similarly, the sheath 40 in Figs. 6A and 6B, upon insertion of the introducer and dilator 62, causing the seam to split along the slit 16 and expand to accommodate larger implements inserted through the sheath 40, the sheath 40 will retain its shape with the slit 16 opened. The sheath 40 may made from a single, homogenous layer of material. It should be understood that the contouring and relative dimensions of both slit 16 and perforations 50 may be varied, and those shown have been shown for exemplary purposes and for purposes of clarity only.

Referring to Figs. 7 and 8, the sheath 40 is part of a complete system 68 for introducing medical implements into the vascular system of a patient. The complete system 68 includes, as shown in Fig. 7, a holder, such as holster 64, an introductory cannula 70, a guide wire 72, the expandable sheath 40, and a stepped dilator 74. The holster 64 includes a mount 76 for supporting the complete system 68 in proximity to the patient during installation into a blood vessel. The complete system 68 may be provided in packaging 80, as shown in Fig. 8, so as to be completely sterile when needed. The packaging 80 has an indicator 84 for identifying that the contents, namely the complete system 68 and its components, are sterile. The package also has a label 82 with indicia for identifying the contents, as well as instructions on usage, warnings, and dates of manufacture, use by, and/or expiration.

It is to be understood that the present invention is not limited to the embodiments described above, but encompasses any and all embodiments within the scope of the following claims.

## Claims

1. An expandable sheath (40) for introducing intravascular devices percutaneously within a vessel of a patient, comprising:
an expandable sheath (40), the sheath (40) defining an elongate flexible tubular member including:
i) a proximal end (42) and an open distal end (44), the expandable sheath (40) being adapted to be positioned in the vessel; the expandable sheath (40) including a wall of substantially uniform thickness, the wall having an outer surface and an inner surface, the wall having a first portion, a transitional portion and a second portion respectively from the proximal end (42) to the distal end (44) and having first, transitional and second inner diameters associated therewith, the first inner diameter being greater than the second inner diameter, the transitional inner diameter being less than the first inner diameter and being greater than the second inner diameter,
ii) the first portion only of the expandable sheath (40) further including indicia (18) for proper orientation and positioning of the expandable sheath (40) upon entry into the vascular vessel of the patient,
iii) an array of perforations (50) formed therethrough, the array of perforations (50) extending only from the second portion to the transitional portion, whereby the array of perforations (50) allow the second and transitional portions to expand, wherein the length of the perforations (50) on the second portion are longer than the length of perforations (50) on the transitional portion thereby the second end provides less resistance to expansion, and
iv) the perforations (50) of the second portion terminate adjacent the open end of the distal end (44) of the sheath (40) thereby not breaching the open end.

2. The expandable sheath (40) for introducing intravascular devices as recited in claim 1, wherein the array of perforations (50) on the sheath (40) defines a linear array of perforations (50).

3. A system for intravascular insertion of a medical implement, comprising:
a cannula (70) for entering a lumen of a vascular vessel of a patient;
a guide wire (72) insertable into the lumen via the cannula (70);
an introducer and dilator (62) adapted to follow the guide wire (72) into the lumen;
the expandable sheath (40) of claim 1 positioned on the introducer and dilator (62); and
an extension collar (76), wherein the medical implement may be received within the vessel by passing through the sheath (40) upon separating the introducer and dilator (62) therefrom.

4. The system for intravascular insertion of a medical implement as recited in claim 3, further comprising a hub (60) attached to the proximal end (42) of the sheath (40), the indicia (18) indicating the alignment of the hub (60) with the sheath (40).

5. The system for intravascular insertion of a medical implement as recited in claim 3, wherein the array of perforations (50) includes a linear array of perforations (50).

6. The system for intravascular insertion of a medical implement as recited in claim 3, whereby the introducer and dilator (62) pass through the first portion of the wall of the sheath (40), and upon entering the transitional portion, and subsequently the second portion of the sheath (40), cause the sheath (40) to expand radially as the introducer and dilator (62) force the perforations (50) to expand along the linear array.

7. The system for intravascular insertion of a medical implement as recited in claim 6, wherein the extension collar (76) includes a releasable connector for releasably engaging the hub (60) of the sheath (40) opposite the first portion, a tubular member extending from the releasable connector, and an end stop opposite the releasable connector.

8. The system for intravascular insertion of a medical implement as recited in claim 7, wherein the extension collar (76) further comprises a secondary releasable connector for releasably engaging a hub (60) of the introducer and dilator (62).

9. A kit including:
the system of claim 3;
a holder for retaining said cannula (70), said guide wire (72), said introducer and dilator (62), said expandable sheath (40) and said extension collar (76); and
sterile packaging (80) for receiving said holder, said cannula (70), said guide wire (72), said introducer and dilator (62), said expandable sheath (40) and said extension collar (76).

10. The kit as recited in claim 9, further comprising a hub (60) attached to the proximal end (42) of the sheath (40).

11. The kit including a system for intravascular insertion of a medical implement as recited in claim 10, wherein the sheath (40) comprises an elongate flexible tubular member adapted for placement percutaneously within the vessel, the tubular member being formed of a polymer material and including a wall of substantially uniform thickness, said wall having an outer surface and an inner surface, said wall having a first portion, a transitional portion and a second portion respectively having first, transitional and second inner diameters associated therewith, the first inner diameter being greater than the second inner diameter, the transitional inner diameter being less than the first inner diameter and being greater than the second inner diameter, whereby, upon placement in the vessel, the sheath (40) is adapted to slidably receive intravascular devices therein.

12. The kit including a system for intravascular insertion of a medical implement as recited in claim 11, wherein the tubular member further includes a linear array of perforations (50) formed therethrough, the linear array of perforations (50) extending from the second portion to the transitional portion, whereby the linear array of perforations (50) allow the second and transitional portions to expand.

13. The kit including a system for intravascular insertion of a medical implement as recited in claim 12, wherein the perforations (50) comprise first and second sets of perforations (50) such that each perforation (50) of said first set is longer than each perforation (50) of said second set, said first set of perforations (50) being formed through the first portion of the wall.

## Patentansprüche

1. Expandierbare Hülle (40) zur perkutanen Einführung intravaskulärer Vorrichtungen in ein Gefäß eines Patienten, umfassend:
eine expandierbare Hülle (40), wobei die Hülle (40) ein längliches flexibles schlauchförmiges Element definiert, enthaltend:
i) ein proximales Ende (42) und ein offenes distales Ende (44), wobei die expandierbare Hülle (40) angepasst ist, um im Gefäß positioniert zu werden; die expandierbare Hülle (40) eine Wand mit im Wesentlichen gleichförmiger Dicke enthält, wobei die Wand eine Außenfläche und eine Innenfläche aufweist, die Wand einen ersten Abschnitt, einen Übergangsabschnitt und einen zweiten Abschnitt jeweils vom proximalen Ende (42) zum distalen Ende (44) aufweist und zugeordnete erste, Übergangs- und zweite Innendurchmesser aufweist, wobei der erste Innendurchmesser größer als der zweite Innendurchmesser ist, der Übergangsinnendurchmesser kleiner als der erste Innendurchmesser und größer als der zweite Innendurchmesser ist,
ii) der erste Abschnitt nur der expandierbaren Hülle (40) ferner Kennzeichen (18) für die richtige Ausrichtung und Positionierung der expandierbaren Hülle (40) nach Eindringen in das vaskuläre Gefäß des Patienten enthält,
iii) eine Anordnung von Perforationen (50), die dadurch geformt sind, wobei sich die Anordnung von Perforationen (50) nur vom zweiten Abschnitt des Übergangsabschnitts erstrecken, wobei die Anordnung von Perforationen (50) dem zweiten und Übergangsabschnitt ermöglichen zu expandieren, wobei die Länge der Perforationen (50) am zweiten Abschnitt länger als die Länge von Perforationen (50) am Übergangsabschnitt ist, wodurch das zweite Ende weniger Widerstand gegenüber Expansion bereitstellt, und
iv) die Perforationen (50) des zweiten Abschnitts angrenzend an das offene Ende des distalen Endes (44) der Hülle (40) enden, wodurch sie das offene Ende nicht brechen.

2. Expandierbare Hülle (40) zur Einführung intravaskulärer Vorrichtungen nach Anspruch 1, wobei die Anordnung von Perforationen (50) an der Hülle (40) eine lineare Anordnung von Perforationen (50) definiert.

3. System zur intravaskulären Einführung eines medizinischen Implantats, umfassend:
eine Kanüle (70) zum Eindringen in ein Lumen eines vaskulären Gefäßes eines Patienten;
einen Führungsdraht (72), der über die Kanüle (70) in das Lumen eingeführt werden kann;
eine Einführ- und Dehnvorrichtung (62), die angepasst ist, um dem Führungsdraht (72) in das Lumen zu folgen;
wobei die expandierbare Hülle (40) nach Anspruch 1 an der Einführ- und Dehnvorrichtung (62) positioniert ist;
und eine Erweiterungsmanschette (76), wobei das medizinische Implantat durch Passieren der Hülle (40) nach Trennung der Einführ- und Dehnvorrichtung (62) davon im Gefäß aufgenommen werden kann.

4. System zur intravaskulären Einführung eines medizinischen Implantats nach Anspruch 3, ferner umfassend eine am proximalen Ende (42) der Hülle (40) angebrachte Nabe (60), wobei die Kennzeichen (18) die Ausrichtung der Nabe (60) auf die Hülle (40) anzeigen.

5. System zur intravaskulären Einführung eines medizinischen Implantats nach Anspruch 3, wobei die Anordnung von Perforationen (50) eine lineare Anordnung von Perforationen (50) enthält.

6. System zur intravaskulären Einführung eines medizinischen Implantats nach Anspruch 3, wobei die Einführ- und Dehnvorrichtung (62) durch den ersten Abschnitt der Wand der Hülle (40) passiert und nach dem Eindringen in den Übergangsabschnitt und anschließend den zweiten Abschnitt der Hülle (40) die Hülle (40) veranlasst, radial zu expandieren, während die Einführ- und Dehnvorrichtung (62) die Perforationen (50) zwingen, entlang der linearen Anordnung zu expandieren.

7. System zur intravaskulären Einführung eines medizinischen Implantats nach Anspruch 6, wobei die Erweiterungsmanschette (76) einen lösbaren Verbinder zur lösbaren Ineingriffnahme der Nabe (60) der Hülle (40) gegenüber dem ersten Abschnitt, wobei sich ein schlauchförmiges Element vom lösbaren Verbinder erstreckt, und einen Endanschlag gegenüber dem lösbaren Verbinder enthält.

8. System zur intravaskulären Einführung eines medizinischen Implantats nach Anspruch 7, wobei die Erweiterungsmanschette (76) ferner einen zweiten lösbaren Verbinder zur lösbaren Ineingriffnahme einer Nabe (60) der Einführ- und Dehnvorrichtung (62) enthält.

9. Set, enthaltend:
das System nach Anspruch 3;
einen Halter zum Zurückhalten der Kanüle (70), den Führungsdraht (72), die Einführ- und Dehnvorrichtung (62), die expandierbare Hülle (40) und die Erweiterungsmanschette (76);
und sterile Verpackung (80) zum Aufnehmen des Halters, der Kanüle (70), des Führungsdrahts (72), der Einführ- und Dehnvorrichtung (62), der expandierbaren Hülle (40) und der Erweiterungsmanschette (76).

10. Set nach Anspruch 9, ferner umfassend eine Nabe (60), die am proximalen Ende (42) der Hülle (40) angebracht ist.

11. Set, enthaltend ein System für die intravaskuläre Einführung eines medizinischen Implantats nach Anspruch 10, wobei die Hülle (40) ein längliches flexibles schlauchförmiges Element umfasst, das zur perkutanen Platzierung im Gefäß angepasst ist, wobei das schlauchförmige Element aus einem Polymermaterial geformt ist und eine Wand mit im Wesentlichen gleichförmiger Dicke enthält, wobei die Wand eine Außenfläche und eine Innenfläche aufweist, wobei die Wand einen ersten Abschnitt, einen Übergangsabschnitt und einen zweiten Abschnitt aufweist, denen jeweils ein erster, Übergangs- und zweiter Innendurchmesser zugeordnet ist, wobei der erste Innendurchmesser größer als der zweite Innendurchmesser ist, der Übergangsinnendurchmesser kleiner als der erste Innendurchmesser und größer als der zweite Innendurchmesser ist, wobei, nach der Platzierung im Gefäß, die Hülle (40) angepasst ist, um die intravaskulären Vorrichtungen darin gleitbar aufzunehmen.

12. Set, enthaltend ein System für die intravaskuläre Einführung eines medizinischen Geräts nach Anspruch 11, wobei das schlauchförmige Element ferner eine lineare Anordnung von dadurch geformten Perforationen (50) enthält, wobei sich die lineare Anordnung von Perforationen (50) vom zweiten Abschnitt zum Übergangsabschnitt erstreckt, wobei die lineare Anordnung von Perforationen (50) dem zweiten und Übergangsabschnitt ermöglicht zu expandieren.

13. Set, enthaltend ein System für die intravaskuläre Einführung eines medizinischen Implantats nach Anspruch 12, wobei die Perforationen (50) erste und zweite Sätze von Perforationen (50) umfassen, so dass jede Perforation (50) des ersten Satzes länger als jede Perforation (50) des zweiten Satzes ist, wobei der erste Satz von Perforationen (50) durch den ersten Abschnitt der Wand geformt ist.

## Revendications

1. Gaine extensible (40) permettant l'introduction de dispositifs intravasculaires par voie percutanée à l'intérieur d'un vaisseau d'un patient, comprenant :
une gaine extensible (40), la gaine (40) définissant un élément tubulaire flexible allongé possédant :
i) une extrémité proximale (42) et une extrémité distale ouverte (44), la gaine extensible (40) étant conçue pour être positionnée dans le vaisseau ; la gaine extensible (40) possédant une paroi d'épaisseur sensiblement uniforme, la paroi possédant une surface externe et une surface interne, la paroi comportant une première partie, une partie de transition et une seconde partie respectivement de l'extrémité proximale (42) à l'extrémité distale (44) et ayant un premier diamètre interne, un diamètre interne de transition et un second diamètre interne associé à celles-ci, le premier diamètre interne étant supérieur au second diamètre interne, le diamètre interne de transition étant inférieur au premier diamètre interne et étant supérieur au second diamètre interne,
ii) la première partie uniquement de la gaine extensible (40) comportant en outre un repère (18) pour la bonne orientation et le bon positionnement de la gaine extensible (40) lors de l'entrée dans le vaisseau vasculaire du patient,
iii) un réseau de perforations (50) formé à travers celle-ci, le réseau de perforations (50) s'étendant uniquement de la seconde partie à la partie de transition, moyennant quoi le réseau de perforations (50) permet à la seconde partie et à la partie de transition de s'étendre, la longueur des perforations (50) sur la seconde partie étant plus longue que la longueur des perforations (50) sur la partie de transition, ce qui permet à la seconde extrémité d'offrir moins de résistance à l'extension, et
iv) les perforations (50) de la seconde partie se terminent à proximité de l'extrémité ouverte de l'extrémité distale (44) de la gaine (40) ce qui permet de ne pas traverser l'extrémité ouverte.

2. Gaine extensible (40) permettant l'introduction de dispositifs intravasculaires selon la revendication 1, ledit réseau de perforations (50) sur la gaine (40) définissant un réseau linéaire de perforations (50).

3. Système permettant l'insertion intravasculaire d'un instrument médical, comprenant :
une canule (70) destinée à pénétrer dans la lumière d'un vaisseau vasculaire d'un patient ;
un fil guide (72) pouvant être inséré dans la lumière via la canule (70) ;
un introducteur et dilatateur (62) conçu pour suivre le fil guide (72) dans la lumière ;
la gaine extensible (40) selon la revendication 1 étant placée sur l'introducteur et récepteur (62) ; et
un collier d'extension (76), ledit instrument médical pouvant être reçu à l'intérieur du vaisseau en passant à travers la gaine (40) lors de la séparation de l'introducteur et dilatateur (62) de celui-ci.

4. Système permettant l'insertion intravasculaire d'un instrument médical selon la revendication 3, comprenant en outre un embout (60) fixé à l'extrémité proximale (42) de la gaine (40), le repère (18) indiquant l'alignement de l'embout (60) avec la gaine (40).

5. Système permettant l'insertion intravasculaire d'un instrument médical selon la revendication 3, ledit réseau de perforations (50) comportant un réseau linéaire de perforations (50).

6. Système permettant l'insertion intravasculaire d'un instrument médical selon la revendication 3, grâce auquel l'introducteur et dilatateur (62) traverse la première partie de la paroi de la gaine (40), et lors de l'entrée de la partie de transition, et par la suite de la seconde partie de la gaine (40), amène la gaine (40) à s'étendre radialement alors que l'introducteur et dilatateur (62) contraint les perforations (50) à s'étendre le long du réseau linéaire.

7. Système permettant l'insertion intravasculaire d'un instrument médical selon la revendication 6, ledit collier d'extension (76) comportant un raccord libérable pour entrer en prise de manière libérable avec l'embout (60) de la gaine (40) face à la première partie, un élément tubulaire s'étendant du raccord libérable, et une butée d'extrémité face au raccord libérable.

8. Système permettant l'insertion intravasculaire d'un instrument médical selon la revendication 7, ledit collier d'extension (76) comportant en outre un raccord libérable secondaire pour entrer en prise de manière libérable avec l'embout (60) de l'introducteur et dilatateur (62).

9. Kit comportant :
le système selon la revendication 3 ;
un support pour retenir ladite canule (70), ledit fil guide (72), ledit introducteur et récepteur (62), ladite gaine extensible (40) et ledit collier d'extension (76) ; et
un emballage stérile (80) pour recevoir ledit support, ladite canule (70), ledit fil guide (72), ledit introducteur et dilatateur (62), ladite gaine extensible (40) et ledit collier d'extension (76).

10. Kit selon la revendication 9, comprenant en outre un embout (60) fixé à l'extrémité proximale (42) de la gaine (40).

11. Kit comportant un système permettant l'insertion intravasculaire d'un instrument médical selon la revendication 10, ladite gaine (40) comprenant un élément tubulaire flexible allongé conçu pour un positionnement par voie percutanée à l'intérieur du vaisseau, l'élément tubulaire étant formé d'un matériau polymère et comportant une paroi d'épaisseur sensiblement uniforme, ladite paroi possédant une surface externe et une surface interne, ladite paroi comportant une première partie, une partie de transition et une seconde partie ayant respectivement un premier diamètre interne, un diamètre interne de transition et un second diamètre interne associé à ceux-ci, le premier diamètre interne étant supérieur au second diamètre interne, le diamètre interne de transition étant inférieur au premier diamètre et étant supérieur au second diamètre interne, moyennant quoi, lors de la disposition dans le vaisseau, la gaine (40) est conçue pour recevoir de manière coulissante des dispositifs intravasculaires en elle.

12. Kit comportant un système permettant l'insertion intravasculaire d'un instrument médical selon la revendication 11, ledit élément tubulaire comportant en outre un réseau linéaire de performations (50) formé à travers lui, le réseau linéaire de perforations (50) s'étendant de la seconde partie à la partie de transition, grâce à quoi le réseau linéaire de perforations (50) permet à la seconde partie et à la partie de transition de s'étendre.

13. Kit comportant un système permettant l'insertion intravasculaire d'un instrument médical selon la revendication 12, lesdites perforations (50) comprenant des premier et second ensembles de perforations (50) de sorte que chaque perforation (50) dudit premier ensemble soit plus longue que chaque perforation (50) dudit second ensemble, ledit premier ensemble de perforations (50) étant formé à travers la première partie de la paroi.
